(19) Europäisches Patentamt
European Patent Office
Office européen des brevets

(11) Veröffentlichungsnummer: **0 069 294**
**B1**

(12) # EUROPÄISCHE PATENTSCHRIFT

(45) Veröffentlichungstag der Patentschrift: **02.01.85**

(21) Anmeldenummer: **82105601.7**

(22) Anmeldetag: **25.06.82**

(51) Int. Cl.³: **C 07 C 103/48**, C 07 C 125/065, C 07 C 149/23, C 07 D 233/56, C 07 D 249/08, C 07 D 307/68, C 07 D 261/18, C 07 D 275/02, C 07 D 277/56, C 07 D 285/00, A 01 N 37/46

(54) **N-substituierte 2-Methylnaphthylamide, Verfahren zu ihrer Herstellung und diese enthaltende Fungizide.**

(30) Priorität: **02.07.81 DE 3126083**

(43) Veröffentlichungstag der Anmeldung: **12.01.83 Patentblatt 83/2**

(45) Bekanntmachung des Hinweises auf die Patenterteilung: **02.01.85 Patentblatt 85/1**

(84) Benannte Vertragsstaaten: **AT BE CH DE FR GB IT LI NL SE**

(56) Entgegenhaltungen:
**EP - A - 0 017 850**
**EP - A - 0 018 510**
**EP - A - 0 029 996**
**DE - A - 3 010 412**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Eicken, Karl, Dr., Waldstrasse 63, D-6706 Wachenheim (DE)**
Erfinder: **Theobald, Hans, Dr., Parkstrasse 2, D-6703 Limburgerhof (DE)**
Erfinder: **Zeeh, Bernd, Dr., Thorwaldsenstrasse 5, D-6700 Ludwigshafen (DE)**
Erfinder: **Pommer, Ernst-Heinrich, Dr., Berliner Platz 7, D-6703 Limburgerhof (DE)**
Erfinder: **Ammermann, Eberhard, Dr., Sachenstrasse 3, D-6700 Ludwigshafen (DE)**

**EP 0 069 294 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue N-substituierte 2-Methylnaphthylamide, Verfahren zu ihrer Herstellung und Fungizide, die diese Verbindungen enthalten.

Es ist bereits bekannt, Zink-ethylen-1,2-bisdithiocarbamat (Chemical Week, 26. Juli 1972, Seite 41) und das N-Trichlormethylthiophthalimid (Chemical Week, 21. Juni 1972, S. 63) als Fungizide in der Landwirtschaft und im Gartenbau zu verwenden. Die genannten Verbindungen sind gute Mittel zur Bekämpfung von pilzlichen Krankheiten.

Jedoch sind diese Fungizide nicht nach erfolgter Infektion verwendbar und ihre Wirkung bei niedrigen Aufwandkonzentrationen genügt nicht den Anforderungen der Praxis.

Es ist ferner bekannt, N-substituierte 2-Methylnaphthylamide und N-acylierte alpha-Naphthylamine als Fungizide zu verwenden (EP-A-0029996 und EP-A-0018510). Außerdem ist es bekannt, N,N-disubstituierte Ethylglycinester (EP-A-0017850 und DE-A-3010412) als Fungizide zu verwenden. Die bekannten Verbindungen haben jedoch nur eine schlechte fungizide Wirkung. Die neuen Verbindungen werden durch sie nicht nahegelegt.

Es wurde nun gefunden, daß neue N-substituierte 2-Methylnaphthylamide der allgemeinen Formel I

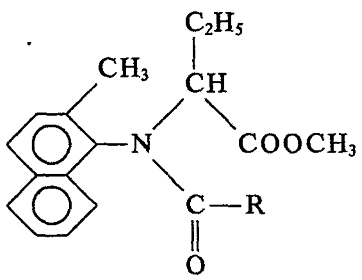

(I)

worin

R einen gegebenenfalls durch Methyl substituierten 2-Furyl-, 3-, 4- oder 5-Isoxazolyl-, 2-, 4- oder 5-Oxazolylrest oder einen gegebenenfalls durch Halogen, Nitro oder Methyl substituierten 3-, 4- oder 5-Isothiazolyl-, 2-, 4- oder 5-Thiazolyl- oder 3-(1,2,4-Oxadiazolyl)-, 4- oder 5-(1,2,3-Thiadiazolyl)-, 3- oder 5-(1,2,4-Thiadiazolyl)-, 3-(1,2,5-Thiadiazolyl)- oder 2-(1,3,4-Thiadiazolyl)-Rest oder einen gegebenenfalls durch Halogen, $C_{1-4}$ Alkoxy, $C_{1-4}$ Alkylthio, 1-Imidazolyl, 1-Pyrazolyl oder 1,2,4-Triazolyl-(1) substituierten $C_1$—$C_5$ Alkylrest oder die Gruppe $-CH_2-Y-R^1$ bedeutet, wobei Y Sauerstoff oder Schwefel ist und $R^1$ einen $C_1$—$C_6$-Alkoxy-ethyl- oder einen $C_1$—$C_6$-Alkoxy-ethoxy-ethylrest bedeutet oder R einen $C_2$—$C_5$ Alkenyl-, $C_2$—$C_5$ Alkinyl-, $C_1$—$C_5$ Alkoxy-, $C_1$—$C_5$ Alkoxycarbonyl- oder einen $C_3$—$C_7$ Cycloalkylrest bedeutet, starke fungizide Eigenschaften aufweisen.

In der Formel I steht R bevorzugt für 2-Furyl, 3- oder 5-Isothiazolyl, 2-, 4- oder 5-Thiazolyl, 3- oder 5-Isoxazolyl, 2-, 4- oder 5-Oxazolyl, 3-(1,2,4,-Oxadiazolyl), 4- oder 5-(1,2,3-Thiadiazolyl), 4-(1,2,5-Thiadiazolyl), Methoxy- und Ethoxymethyl, Methylthio- und Ethylthiomethyl, Methoxyethyl, 1-Imidazolylmethyl, 1-Pyrazolylmethyl, 1-(1,2,4-Triazolyl)-methyl, Methyl, Ethyl, Propyl, Chlormethyl, Brommethyl, 1-Chlorethyl, 2-Chlorethyl, 1-Chlorpropyl, 3-Chlorpropyl, 4-Chlorbutyl, Vinyl, Propenyl, Ethynyl, Methoxy, Ethoxy, Methoxycarbonyl, Ethoxycarbonyl, Cyclopropyl und Cyclohexyl.

$R^1$ steht bevorzugt für Methoxyethoxy, Ethoxyethoxy, Methoxyethoxy-ethoxy und Butoxyethoxyethoxy.

Die neuen N-substituierten 2-Methylnaphthylamide der Formel I besitzen ein chirales Kohlenstoffatom im alpha-C-Atom der Buttersäurestruktur der Amide und je nach der Beschaffenheit von R, noch weitere Chiralitätszentren im Rest R. Nach üblichen Methoden können die optisch reinen Enantiomeren bzw. die Diastereomeren erhalten werden. Die vorliegende Erfindung erfaßt auch diese Verbindungen in reiner Form oder als Mischungen. Als Fungizide sind sowohl die reinen Enantiomeren bzw. die einheitlichen Diastereomeren als auch die bei der Synthese üblicherweise anfallenden Mischungen wirksam.

Weiterhin wurde gefunden, daß man die neuen Verbindungen der allgemeinen Formel I erhält, wenn man ein 2-Methylnaphthylamin der Formel II

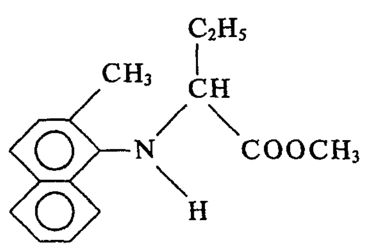

(II)

a) mit einem Säurehalogenid der Formel III

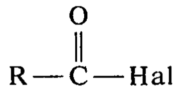

(III)

oder

b) mit einem Säureanhydrid der Formel IV

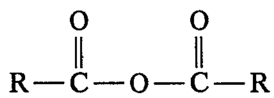

(IV)

in welcher
R die oben genannte Bedeutung hat und Hal Chlor oder Brom bedeutet, gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers, bei Temperaturen zwischen −10 und 100°C umsetzt. Diese Umsetzung wird bevorzugt. Als bevorzugte, gegenüber den Reaktionsteilnehmern inerte Lösungs- oder Verdünnungsmittel werden z. B. aliphatische oder aromatische Kohlenwasserstoffe wie Pentan, Cyclohexan, Petrolether, Benzol, Toluol oder Xylole; Halogenkohlenwasserstoffe, beispielsweise Methylenchlorid, Chloroform, 1,2-Dichlorethan, Chlorbenzol; Ketone wie Aceton und Methylethylketon; Ether wie Diethylether, Dimethoxyethan, Tetrahydrofuran oder Dioxan; Ester wie Essigsäureethylester; Nitrile wie Acetonitril; Sulfoxide wie Dimethylsulfoxid oder entsprechende Gemische verwendet.

Geeignete anorganische oder organische Basen, die gegebenenfalls auch als säurebindende Mittel bei der Reaktion verwendet werden können, sind beispielsweise Alkali- und Erdalkalicarbonate wie Natrium- oder Kaliumbicarbonat, Natrium- oder Kaliumcarbonat, Calciumcarbonat; Borate wie Natriumborat; Phosphate wie Natrium- oder Kaliumdi- oder -triphosphat oder Amine wie Triethylamin, N,N-Dimethylanilin, N,N-Dimethylcyclohexylamin, N-Methylpiperidin oder Pyridin. Es können aber auch andere übliche Basen verwendet werden.

Das Herstellungsverfahren a) kann auch ohne säurebindende Mittel durchgeführt werden, wobei in einigen Fällen das Durchleiten von trockenem Stickstoff zur Vertreibung des gebildeten Halogenwasserstoffs angezeigt ist.

Als Reaktionsbeschleuniger kommen vorzugsweise Metallhalogenide wie Natriumbromid oder Kaliumjodid, Azole wie Imidazol oder 1,2,4-Triazol oder Pyridine wie 4-Dimethylaminopyridin in Frage.

Die erfindungsgemäßen Umsetzungen erfolgen beispielsweise bei Temperaturen zwischen −10 und +100°C, vorzugsweise zwischen 0 und +80°C, durcklos oder unter Druck, kontinuierlich oder diskontinuierlich.

Es wurde weiterhin gefunden, daß man die Verbindung der allgemeinen Formel II erhält, wenn man

a) 2-Methylnaphthylamin mit dem 2-Ketobuttersäuremethylester der Formel V

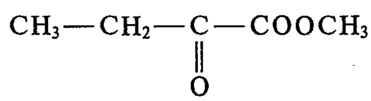

(V)

umsetzt und
die als Umsetzungsprodukt erhaltene Schiffsche Base hydriert, z. B. mit komplexen Metallhydriden oder auf katalytischem Weg mit Wasserstoff, oder

b) 2-Methylnaphthylamin mit 2-Chlor- oder 2-Brombuttersäuremethylester gegebenenfalls in Gegenwart eines Lösungs- oder Verdünnungsmittels, gegebenenfalls in Gegenwart einer anorganischen Base und gegebenenfalls unter Zusatz eines Reaktionsbeschleunigers bei Temperaturen zwischen 40 und 140°C umsetzt.

Die Herstellung der Schiff'schen Base wird beispielsweise so durchgeführt, daß man 1 Mol des 2-Methylnaphthylamins mit 0,9 bis 1,5 Mol des 2-Ketobuttersäuremethylesters der Formel V in einem Lösungsmittel, gegebenenfalls unter Zusatz eines sauren Katalysators, umsetzt und bei einer Temperatur von 40 bis 200°C, vorzugsweise 50 bis 120°C Wasser durch Destillation abtrennt. Zweckmäßig verwendet man unter den Reaktionsbedingungen inerte und gleichzeitig mit Wasser Azeotrope bildende Lösungsmittel für die Umsetzung. Als Lösungsmittel kommen z. B. in Frage aromatische Kohlenwasserstoffe, beispielsweise Benzol, Toluol, Ethylbenzol, o-, m-, p-Xylol, Isopropylbenzol, Methylnaphthalin; aliphatische oder cycloaliphatische Kohlenwasserstoffe, beispielsweise Heptan, Nonan, Pinan, Benzinfraktionen innerhalb eines Siedepunktintervalls von 70 bis 190°C, Cyclohexan, Methylcyclohexan, Dekalin, Ligroin, 2,2,4-Trimethylpentan, 2,2,3-Trimethylpentan, 2,3,3-Trimethylpentan, Octan und entsprechende Gemische.

Für die Hydrierung eignen sich sowohl die Reduktion mit komplexen Hydriden wie $NaBH_4$ als auch

katalytische Hydrierung mit Wasserstoff.

Die Reduktion mit Natriumborhydrid wird im allgemeinen so durchgeführt, daß man in einem Lösungsmittel die Schiff'sche Base mit 0,2 Mol bis 1 Mol Natriumborhydrid pro Mol Schiff'sche Base bei einer Temperatur zwischen −20 und +40°C umsetzt.

Bei der katalytischen Hydrierung werden dem Reaktionsgemisch am Anfang und im Verlauf der Umsetzung solche Mengen an Wasserstoff zugeführt, daß sich bei der Umsetzungstemperatur stets ein entsprechender Reaktionsdruck, zweckmäßig zwischen 150 und 300 bar, einstellt. Die Umsetzung wird im allgemeinen bei einer Temperatur von 20 bis 200°C, vorzugsweise von 25 bis 160°C, diskontinuierlich oder kontinuierlich durchgeführt. Zur entsprechenden Druckeinstellung können auch inerte Gase wie Stickstoff verwendet werden.

Für beide Verfahrensvarianten der Hydrierung eignen sich als Lösungs- oder Verdünnungsmittel besonders gut Alkanole und Cycloalkanole, z. B. n-Propanol, Isopropanol, n-Butanol, Isobutanol, Glykol, Ethylenglykolmonomethylether, Glycerin, Amylalkohol, Cyclohexanol, 2-Methyl-4-pentanol, 2-Ethylhexanol und insbesondere Methanol, Ethanol; cyclische Ether wie Tetrahydrofuran und Dioxan.

Zur katalytischen Hydrierung wird der Katalysator in der Regel in einer Menge von 5 bis 30 Gew.-%, bezogen auf die Schiff'sche Base, verwendet. Er kann im Gemisch mit einem für die Umsetzung geeigneten Trägermaterial, z. B. Siliciumdioxid, zur Anwendung gelangen, wobei zweckmäßig die Menge des Katalysators 10 bis 40 Gew.-% des Gemisches von Katalysator und Träger beträgt.

Zweckmäßig verwendet man Kupferchromitkatalysatoren, z. B. Kupferchromoxidkatalysatoren wie die von H. Adkins (vgl. Houben-Weyl, Methoden der organischen Chemie, Band 4/2, Seiten 180 bis 183 und J. Applied Chem. 5, 289–295 (1955)) verwendeten Kupferchromite. Sie enthalten beispielsweise Kupfer-Chrom-Spinell ($CuCr_2O_4$) oder Gemische im Verhältni 5 CuO : 4 $Cr_2O_3$ bzw. gehen von solchen Verbindungen aus und können noch andere Oxide, hauptsächlich die der Erdalkalimetalle wie Magnesium, Calcium oder Barium enthalten.

Folgende Verfahrensbeschreibungen erläutern die Herstellung von 2-Methylnaphthylamin der Formel II:

$a_1$) Schiff'sche Base des 1-Amino-2-methylnaphthalins

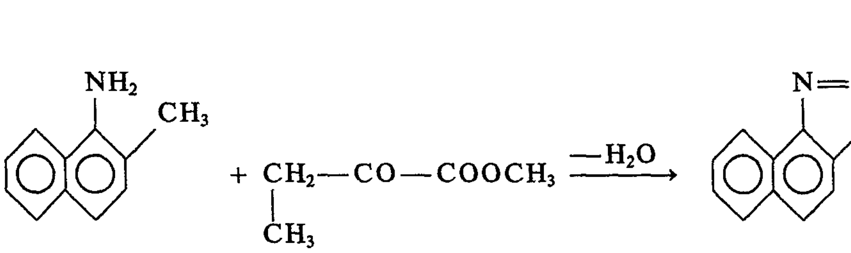


(1)

314 g 1-Amino-2-methylnaphthalin (2 Mol), 232 g 2-Ketobuttersäuremethylester (2 Mol) und 0,4 g p-Toluolsulfonsäure werden in 1000 ml Cyclohexan für 4 Stunden am Rückfluß erhitzt bis 36 g Wasser azeotrop abdestilliert und aus dem Destillat abgetrennt sind. Dann wird das Cyclohexan im Vakuum abdestilliert und der Rückstand direkt weiter umgesetzt.

Ausbeute: 494,7 g (97% d. Th.) Schiff'sche Base.

$a_2$) Hydrierung der Schiff'schen Base
Katalytische Hydrierung

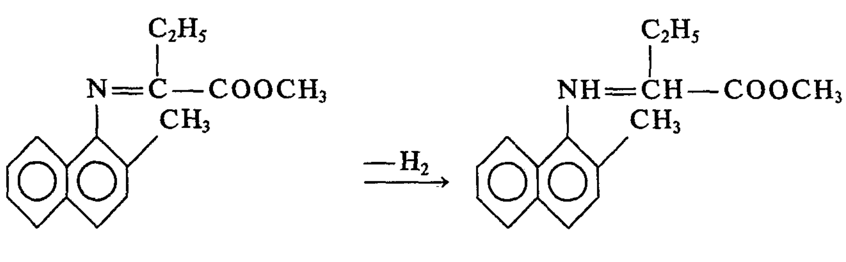


(2)

b) Direkte Alkylierung von 1-Amino-2-methylnaphthalin

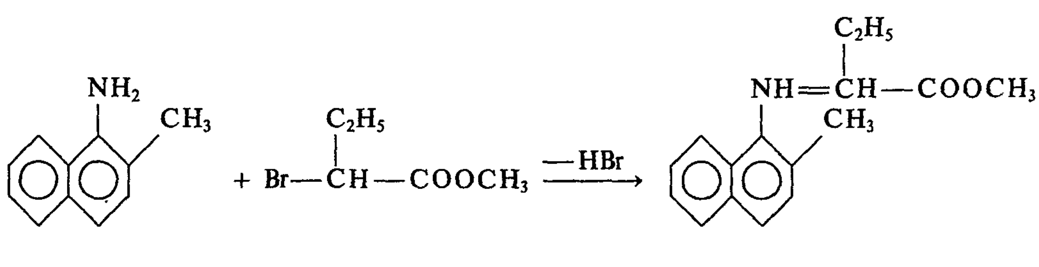


(3)

144,2 g 1-Amino-2-methylnaphthalin (0,92 Mol) werden mit 90,2 g (1,08 Mol) Natriumhydrogencarbonat und 507 g (2,8 Mol) 2-Brombuttersäuremethylester bei 120 bis 125°C 18 Stunden gerührt. Nach dem Abkühlen wird der Niederschlag abgesaugt und das Filtrat im Vakuum eingeengt. Der Rückstand wird im Vakuum destilliert. Man erhält 204,3 g (79,5% d. Th.) 2-(2-Methyl)-1-naphthylamino)-buttersäuremethylester als farbloses Öl. Sdp.: 148—150°C/ 0,25 mbar und $n_D^{25} = 1,5779$.

Ein Hydrierautoklav mit einem Volumen von 1 l wird mit 200 g der Schiff'schen Base aus 1-Amino-2-methylnaphthalin und 2-Ketobuttersäuremethylester, die in 500 Teilen Tetrahydrofuran gelöst sind, und 15 Teilen Adkins-Katalysator (Kupferchromit in pulverisierter Form) gefüllt. Anschließend wird der Autoklav auf 150°C erhitzt und Wasserstoff bis zum Erreichen eines Druckes von 200 bar aufgepreßt. Sobald die Wasserstoffaufnahme beendet und ein konstanter Druck erreicht ist (nach ca. 9 Stunden), wird abgekühlt, der Katalysator abgesaugt und das Filtrat im Vakuum destilliert.

Man erhält 169,3 g (84% d. Th.) 2-(2-Methyl-1-naphthylamino)-buttersäuremethylester als farbloses Öl vom Sdp. 147 bis 150°C/0,25 mbar.

Die Herstellung der neuen Verbindungen der Formel I wird durch folgende Beispiele erläutert:

Beispiel 1

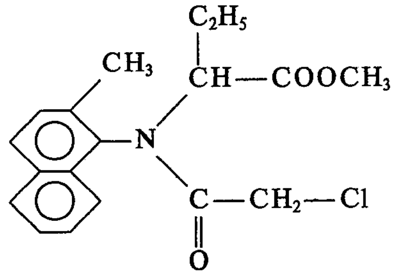

Einer Lösung von 51,4 g (0,2 Mol) 2-(2-Methyl-1-naphthylamino)-buttersäuremethylester in 250 ml trockenem Toluol wurden 27,2 g (0,24 Mol) Chloracetylchlorid zwischen +15 und +30°C zugetropft. Das Gemisch wurde bei 80°C 8 Stunden nachgerührt. Dabei wurde der entstandene Chlorwasserstoff in einem schwachen Stickstoffstrom ständig aus dem Reaktionsgemisch vertrieben. Nach Abkühlen auf 20°C wurde das Gemisch 0,5 Stunden mit der Lösung von 35 g Natriumhydrogencarbonat in 500 ml Wasser gerührt, die organische Phase abgetrennt, über $Na_2SO_4$ getrocknet, mit Kohle entfärbt und im Vakuum eingedampft. Der verbleibende harzige Rückstand wurde 4 Stunden bei 50°C und 0,2 mbar getrocknet. Man erhielt 61,5 g (92,2% d. Th.) analysenreinen 2-(2-Methyl-1-(N-chloractyl)-naphthylamino)-buttersäuremethylester als hellbraunes Harz (Verbindung Nr. 1).

IR-Spektrum (KBr): 3045, 2970, 2940, 1731, 1665, 1422, 1345, 1240, 1190, 1166, 980, 813, 780, und 746 $cm^{-1}$.

Beispiel 2

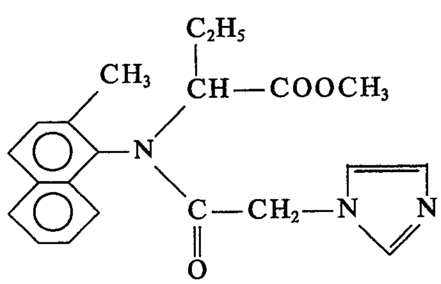

12 g (0,036 Mol) 2-(2-Methyl-1-(N-chloracetyl)-naphthylamino)-buttersäuremethylester (Beispiel 1) wurden in 60 ml trockenem N,N-Dimethylformamid gelöst und mit 7,3 g (0,108 Mol) Imidazol 10 Stunden bei 70°C gerührt. Das Gemisch wurde im Vakuum eingeengt und der Rückstand mit 150 ml Methylenchlorid und 50 ml Wasser ausgeschüttelt. Die organische Schicht wurde abgetrennt, zweimal mit je 50 ml Wasser gewaschen, über Natriumsulfat getrocknet und im Vakuum eingeengt. Man erhielt 8 g (61,6% d. Th.) 2-[2-Methyl-1-(N-(1-Imidazolylacetyl))-naphthyl-amino]-buttersäuremethylester als gelbliches Harz (Verbindung Nr. 2).

Ber. für $C_{12}H_{23}N_3O_3$: C 69,04 H 6,30 N 11,50
Gef. C 68,6 H 6,1 N 11,3

In entsprechender Weise wurden die folgenden Verbindungen hergestellt.

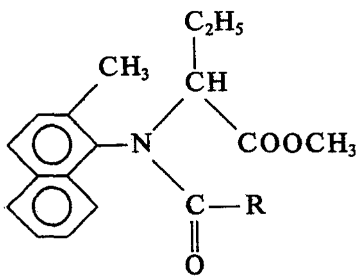

| Nr. | R | Physikal. Konstante oder IR-Spektrum [$cm^{-1}$] [KBr] | |
|---|---|---|---|
| 3 | $-CH_2-CH_3$ | Harz | |
| 4 | $-CH_2-Br$ | Öl | 3030, 2970, 2940, 1732, 1650, 1416, 1358, 1200, 1130, 1095, 813, 784, 742. |
| 5 | 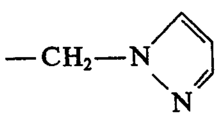 | Harz | |
| 6 | 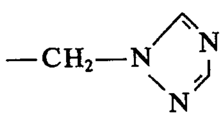 | Harz | |
| 7 | $-CH_2-O-CH_3$ | Öl | 3028, 1730, 1668, 1450, 1382, 1288, 1190, 1123, 995, 930, 814, 788, 748. |
| 8 | $-CH_2-S-CH_3$ | Öl | 3040, 1730, 1648, 1440, 1362, 1212, 1185, 1102, 980, 812, 980, 746. |
| 9 | $-CH-O-CH_2-CH_2-O-CH_3$ | Öl | $n_D^{25}$ = 1,5555 |
| 10 | $-CH_2-(O-CH_2-CH_2)_2O-CH_3$ | Öl | $n_D^{25}$ = 1,5475 |
| 11 | $-CH_2-(O-CH_2-CH_2)_2O-C_4H_9-n$ | Öl | $n_D^{25}$ = 1,5324 |
| 12 | 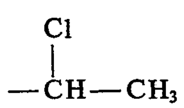 | Öl | 3060, 2975, 1743, 1671, 1395, 1371, 1240, 1200, 1175, 1067, 974, 821, 750. |
| 13 | $-CH_2-CH_2-Cl$ | Öl | 3050, 2970, 2940, 1732, 1652, 1421, 1387, 1360, 1250, 1190, 1168, 1000, 915, 814, 790, 746. |
| 14 | $-CH_2-CH_2-CH_2-Cl$ | Öl | 3050, 2968, 2945, 1732, 1650, 1435, 1385, 1327, 1290, 1250, 1192, 1168, 872, 814, 790, 747. |

Fortsetzung

| Nr. | R | Physikal. Konstante oder IR-Spektrum [$cm^{-1}$] [KBr] | |
|---|---|---|---|
| 15 | $-CH{=}CH-CH_3$ | Harz | 3048, 3010, 2970, 2940, 1732, 1655, 1620, 1432, 1342, 1235, 1190, 958, 900, 813, 790. |
| 16 | Cyclopropyl | Harz | 3052, 3008, 2970, 2945, 1746, 1658, 1410, 1280, 1255, 1200, 1175, 955, 815, 790, 750. |
| 17 | Cyclohexyl | Harz | |
| 18 | $-O-CH_3$ | Harz | |
| 19 | $-O-C_2H_5$ | Harz | |
| 20 | 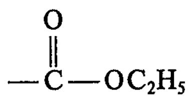 | Harz | |
| 21 | 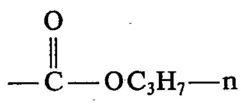 | Harz | |
| 22 | 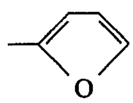 | Schmp. | 128–130°C |
| 23 | 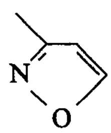 | Harz | 3155, 3120, 3050, 2970, 2948, 1732, 1650, 1450, 1328, 1190, 1170, 982, 748. |
| 24 | 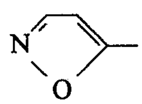 | Harz | 3158, 3100, 3048, 2970, 2945, 1740, 1650, 1512, 1505, 1460, 1320, 1200, 986, 916, 817, 790, 750. |
| 25 | 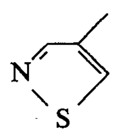 | Öl | |
| 26 | 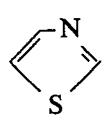 | Öl | |
| 27 | 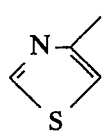 | Öl | |
| 28 | 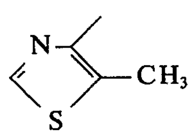 | Harz | |

Fortsetzung

| Nr. | R | Physikal. Konstante oder IR-Spektrum [$cm^{-1}$] [KBr] |
|---|---|---|
| 29 | 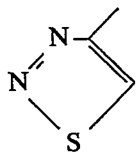 | Harz |
| 30 | 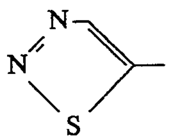 | Öl |
| 31 | 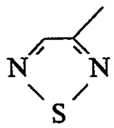 | Harz |

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben, Erysiphe graminis an Getreide, Uncinula necator an Reben, Podophaera leucotricha an Äpfeln, Sphaerotheca fuliginea an Rosen, Erysiphe cichoriacearum an Gurken. Die fungiziden Mittel enthalten 0,1 bis 95% (Gewichtsprozent) Wirkstoff, vorzugsweise 0,5 bis 90%. Die Aufwandmengen liegen je nach Art des gewünschten Effektes zwischen 0,1 un 5 kg Wirkstoff je ha. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen.

Darüber hinaus sind viele der neuen Verbindungen systemisch wirksam, so daß über die Wurzelbehandlung auch ein Schutz oberirdischer Pflanzenteile möglich ist.

Die erfindungsgemäßen Wirkstoffe können auch zusammen mit anderen Wirkstoffen, z. B. Herbiziden, Isektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d. h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt:

Manganethylenbisdithiocarbamat
Mangan-Zinkethylenbisdithiocarbamat
Ammoniak-Komplex von Zink-(N,N-ethylen-bis-dithiocarbamat)
N-Trichlormethylthio-tetrahydrophthalimid
N-Trichlormethylthio-phthalimid
5-Ethoxy-3-trichlormethyl-1,2,3-thiadiazol
2-Methoxycarbonylamino-benzimidazol
2-Rhodanmethylthiobenzthiazol
1,4-Dichlor-2,5-dimethoxybenzol
2,3-Dichlor-6-methyl-1,4-oxathün-5-carbonsäureanilid
2-Methyl-5,6-dihydro-4-H-pyran-3-carbonsäure-anilid
2,4,5-Trimethyl-furan-3-carbonsäureanilid
2-Methyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Wirkstoffen kombiniert werden können sind beispielsweise:

Dithiocarbamate und deren Derivate, wie
- Ferridimethyldithiocarbamat
- Zinkdimethyldithiocarbamat
- Zinkethylenbisdithiocarbamat
- Tetramethylthiuramidsulfide
- Zink-(N,N-propylen-bis-dithiocarbamat)
- Ammoniak-Komplex von Zink-(N,N'-propylen-bis-dithiocarbamat) und N,N'-Polypropylen-bis-(thiocarbamoyl)-disulfid

Nitroderivate, wie
- Dinitro-(1-methylheptyl)-phenylcrotonat
- 2-sec.-Butyl-4,6-dinitrophenyl-3,3-dimethylacrylat
- 2-sec.-Butyl-4,6-dinitrophenyl-isopropylcarbonat

heterocyclische Strukturen, wie
- 2-Heptadecyl-2-imidazolin-acetat
- 2,4-Dichlor-6-(o-chloranilino)-s-triazin
- 0,0-Diethyl-phthalimidophosphonothioat
- 5-Amino-1-(bis-(dimethylamino)-phosphinyl)-3-phenyl-1,2,4-triazol)
- 2,3-Dicyano-1,4-dithiaanthrachinon
- 2-Thio-1,3-dithio-(4,5-b)-chinoxalin
- 1-(Butylcarbamoyl)-2-benzimidazol-carbaminsäuremethyl-ester
- 4-(2-Chlorphenylhydrazono)-3-methyl-5-isoxazolon
- Pyridin-2-thio-1-oxid
- 8-Hydroxychinolin bzw. dessen Kupfersalz
- 2,3-Dihydro-5-carboxanilido-6-methyl-1,4-oxathiin-4,4-dioxid
- 2,3-Dihydro-5-carboxanilido-6-methyl-1,4-oxathiin
- 2-(Furyl-(2))-benzimidazol
- Piperazin-1,4-di-yl-bis-(1-(2,2,2-trichlor-ethyl)-formamid
- 2-(Thiazolyl-(4)-benzimidazol
- 5-Butyl-2-dimethylamino-4-hydroxy-6-methyl-pyrimidin
- Bis-(p-chlorphenyl)-3-pyridinmethanol
- 1,2-Bis-(3-ethoxycarbonyl-2-thioureido)-benzol
- 1,2-Bis-(3-methoxycarbonyl-2-thioureido)-benzol

sowie verschiedene Fungizide, wie
- Dodecylguanidinacetat
- 3-(3-(3,5-Dimethyl-2-oxycyclohexyl)-2-hydroxyethyl)-glutarimid
- Hexachlorbenzol
- N-Dichlorfluormethylthio-N',N'-dimethyl-N-phenyl-schwefelsäurediamid
- 2,5-Dimethyl-furan-3-carbonsäureanilid
- 2-Methyl-benzoesäure-anilid
- 2-Jod-benzoesäure-anilid
- 1-(3,4-Dichloranilino)-1-formylamino-2,2,2-trichlorethan
- 2,6-Dimethyl-N-tridecyl-morpholin bzw. dessen Salze
- 2,6-Dimethyl-N-cyclododecyl-morpholin bzw. dessen Salze
- 1-(4-Chlorphenoxy)-3,3-dimethyl-1-(1 H-1,2,4-triazol-1-yl)-2-butanon
- 1-(4-Chlorphenoxy)-3,3-dimethyl-1-(1 H-1,2,4-triazol-1-yl)-2-butanol
- $\alpha$-(2-Chlorphenyl)-$\alpha$-(4-chlorphenyl)-5-pyrimidin-methanol.

Die neuen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten, durch Versprühen, Vernebeln, Verstäuben, Verstreuen, Beizen oder Gießen angewendet. Die Aufwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate z. B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Haft-, Netz-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind. Als oberflächenaktive Stoffe kommen in Betracht:

Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehle, Cellulosepulver und andere feste Trägerstoffe.

Für die folgenden Versuche wurden als bekannte Vergleichswirkstoffe die folgenden Verbindungen verwendet.

N-Trichlormethylthio-phthalimid (Verbindung A)
Zink-ethylen-1,2-bis-dithiocarbamat (Verbindung B).

## Versuch 1

### Wirksamkeit gegen Plasmopara viticola

Blätter von Topfreben der Sorte »Müller-Thurgau« wurden mit wäßriger Spritzbrühe, die 80% (Gew.-%) Wirkstoff und 20% Emulgiermittel in der Trockensubstanz enthält, besprüht. Um die Wirkungsdauer der Wirkstoffe beurteilen zu können, wurden die Pflanzen nach dem Antrocknen des Spritzbelages 10 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 16 Stunden in einer wasserdampfgesättigten Kammer bei 24°C und anschließend für 8 Tage in einem Gewächshaus mit Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruches auf den Blattunterseiten. Beispielsweise zeigten die Wirkstoffe 9 und 10 bei Anwendung einer 0,025%igen Wirkstoffbrühe eine bessere fungizide Wirkung (beispielsweise 100%) als das bekannte Vergleichsmittel A (beispielsweise 90%).

## Versuch 2

### Wirksamkeit gegen Phytophthora infestans an Tomaten

Blätter von Topfpflanzen der Sorte »Große Fleischtomate« wurden mit wäßriger Spritzbrühe, die 80% Wirkstoff und 20% Emulgiermittel in der Trockensubstanz enthält, besprüht. Nach dem Austrocknen des Spritzbelages wurden die Blätter mit einer Zoosporenaufschwemmung des Pilzes Phytophthora infestans infiziert. Die Pflanzen wurden dann in einer wasserdampfgesättigten Kammer bei Temperaturen zwischen 16 und 18°C aufgestellt. Nach 5 Tagen hatte sich die Krankheit auf den unbehandelten, jedoch infizierten Kontrollpflanzen so stark entwickelt, daß die fungizide Wirksamkeit der Substanzen beurteilt werden konnte.

Beispielsweise zeigten die Wirkstoffe 1, 7, 9, 13, 14, 16, 22, 23 und 24 bei der Anwendung als 0,025%ige Wirkstoffbrühe eine bessere fungizide Wirkung (beispielsweise 100%) als der bekannte Vergleichswirkstoff B (beispielsweise 80%).

Beispiele für Zubereitungen sind:

I. Man vermischt 90 Gew.-Teile der Verbindung 1 mit 10 Gew.-Teilen N-Methyl-alpha-pyrrolidon und erhält eine Lösung, die zur Anwendung in Form kleinster Tropfen geeignet ist.

II. 20 Gew.-Teile der Verbindung 7 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-mono-ethanolamin, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung 7 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung 9 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung 10 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung 13 werden mit 97 Gew.-Teilen feinteiligem Kaolin innig vermischt. Man erhält auf diese Weise ein Stäubemittel, das 3 Gew.-% des Wirkstoffs enthält.

VII. 30 Gew.-Teile der Verbindung 23 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung 24 werden mit 10 Teilen Natriumsalz eines Phenolsulfonsäureharnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung 16 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoffformaldehyd-Kondensats und 68 Teilen eines paraffinischen Mineralöls innig vermischt. Man erhält eine stabile ölige Dispersion.

**Patentansprüche für die Vertragsstaaten: BE, CH, DE, FR, GB, IT, LI, NL, SE**

1. N-Substituiertes 2-Methylnaphthylamid der allgemeinen Formel I

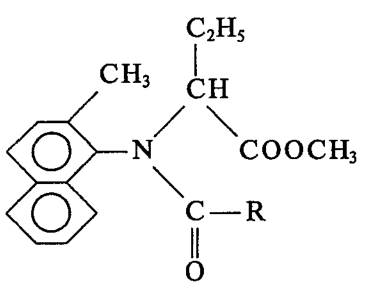

(I)

worin,
R einen gegebenenfalls durch Methyl substituierten 2-Furyl-, 3-, 4- oder 5-Isoxazolyl-, 2-, 4- oder 5-Oxazolylrest oder einen gegebenenfalls durch Halogen, Nitro oder Methyl substituierten 3-, 4- oder 5-Isothiazolyl-, 2-, 4- oder 5-Thiazolyl- oder 3-(1,2,4-Oxadiazolyl)-, 3- oder 5-(1,2,4-Thiadiazolyl)-, 3-(1,2,5-Thiadiazolyl)- oder 2-(1,3,4-Thiadiazolyl)-Rest oder einen gegebenenfalls durch Halogen, $C_{1-4}$ Alkoxy, $C_{1-4}$ Alkylthio, 1-Imidazolyl, 1-Pyrazolyl oder 1,2,4-Triazolyl-(1) substituierten $C_1$—$C_5$ Alkylrest oder die Gruppe $-CH_2-Y-R^1$ bedeutet, wobei Y Sauerstoff oder Schwefel ist und $R^1$ einen $C_1$—$C_6$-Alkoxy-ethyl- oder einen $C_1$—$C_6$ Alkoxy-ethoxy-ethylrest bedeutet oder R einen $C_2$—$C_5$-Alkenyl-,

$C_2$—$C_5$ Alkinyl-, $C_1$—$C_5$ Alkoxy-, $C_1$—$C_5$ Alkoxycarbonyl- oder einen $C_3$—$C_7$ Cycloalkylrest bedeutet.

2. Fungizid, enthaltend ein N-substituiertes 2-Methylnaphthylamid gemäß Anspruch 1.

3. Fungizid, enthaltend einen festen oder flüssigen Trägerstoff und ein N-substiuiertes 2-Methylnaphthylamid gemäß Anspruch 1.

4. Verfahren zur Herstellung eines Fungizids, dadurch gekennzeichnet, daß man einen festen oder flüssigen Trägerstoff vermischt mit einem N-substituierten 2-Methylnaphthylamid gemäß Anspruch 1.

5. Verfahren zur Bekämpfung von Pilzen, dadurch gekennzeichnet, daß man die Pilze oder die vor Pilzbefall zu schützenden Gegenstände behandelt mit einem N-substituierten 2-Methylnaphthylamid gemäß Anspruch 1.

6. Verfahren zur Herstellung eines N-substituierten 2-Methylnaphthylamids gemäß Anspruch 1, dadurch gekennzeichnet, daß man ein 2-Methylnaphthylamid der Formel II

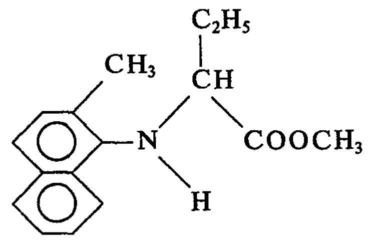

(II)

a) mit einem Säurehalogenid der Formel III

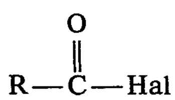

(III)

oder

b) mit einem Säureanhydrid der Formel IV

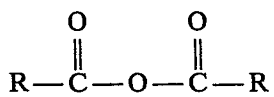

(IV)

worin
R die in Anspruch 1 angegebene Bedeutung hat und Hal Chlor oder Brom bedeutet, gegebenenfalls in Gegenwart eines Lösungsmittels, gegebenenfalls unter Zusatz einer anorganischen oder organischen Base und gegebenenfalls unter Zusatz eines Reaktionsbeschleunigers bei Temperaturen zwischen —10 und 100°C umsetzt.

7. N-Substituiertes 2-Methylnaphthylamid gemäß Anspruch 1, dadurch gekennzeichnet, daß R 2-Furyl, 3- oder 5-Isothiazolyl, 2-, 4- oder 5-Thiazolyl, 3- oder 5-Isoxyzolyl, 2-, 4- oder 5-Oxazolyl, 3-(1,2,4-Oxadiazolyl), 4- oder 5-(1,2,3-Thiadiazolyl), 4-(1,2,5-Thiadiazolyl), Methoxy- oder Ethoxymethyl, Methylthio- oder Ethylthiomethyl, Methoxyethyl, 1-Imidazolylmethyl, 1-Pyrazolylmethyl, 1-(1,2,4-Triazolyl)-methyl, Methyl, Ethyl, n-Propyl, Chlormethyl, Brommethyl, 1-Chlorethyl, 2-Chlorethyl, 1-Chlorpropyl, 3-Chlorpropyl, 4-Chlorbutyl, Vinyl, Propenyl, Ethinyl, Methoxy, Ethoxy, Propoxy, Methoxycarbonyl, Ethoxycarbonyl, Propoxycarbonyl, Cyclopropyl und Cyclohexyl und $R^1$-Y-Methoxyethoxy, Ethoxyethoxy, Methoxyethoxyethoxy und Butoxyethoxyethoxy bedeutet.

8. Fungizid, enthaltend ein N-substiuiertes 2-Naphthylamid definiert wie in Anspruch 1, dadurch gekennzeichnet, daß R 2-Furyl, 3- oder 5-Isothiazolyl, 2-, 4- oder 5-Thiazolyl, 3- oder 5-Isoxazolyl, 2-, 4- oder 5-Oxazolyl, 3-(1,2,4-Oxadiazolyl), 4- oder 5-(1,2,3-Thiadiazolyl), 4-(1,2,5-Thiadiazolyl), Methoxy- oder Ethoxymethyl, Methylthio- oder Ethylthiomethyl, Methoxyethyl, 1-Imidazolylmethyl, 1-Pyrazolylmethyl, 1-(1,2,4-Triazolyl)-methyl, Methyl, Ethyl, n-Propyl, Chlormethyl, Brommethyl, 1-Chlorethyl, 2-Chlorethyl, 1-Chlorpropyl, 3-Chlorpropyl, 4-Chlorbutyl, Vinyl, Propenyl, Ethinyl, Methoxy, Ethoxy, Propoxy, Methoxycarbonyl, Ethoxycarbonyl, Propoxycarbonyl, Cyclopropyl und Cyclohexyl und $R^1$-Y-Methoxyethoxy, Ethoxyethoxy, Methoxyethoxyethoxy und Butoxyethoxyethoxy bedeutet.

**Patentansprüche für den Vertragsstaat: AT**

1. Fungizid, enthaltend ein N-substituiertes 2-Methylnaphthylamid der allgemeinen Formel I

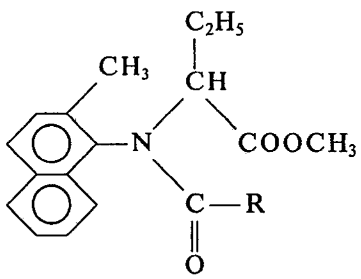

(I)

worin
R einen gegebenenfalls durch Methyl substituierten 2-Furyl-, 3-, 4- oder 5-Isoxazolyl-, 2-, 4- oder 5-Oxazolylrest oder einen gegebenenfalls durch Halogen, Nitro oder Methyl substituierten 3-, 4- oder 5-Isothiazolyl-, 2-, 4- oder 5-Thiazolyl- oder 3-(1,2,4-Oxadiazolyl)-, 3- oder 5-(1,2,4-Thiadiazolyl)-, 3-(1,2,5-Thiadiazolyl)- oder 2-(1,3,4-Thiadiazolyl)-Rest oder einen gegebenenfalls durch Halogen, $C_{1-4}$- Alkoxy, $C_{1-5}$ Alkylthio, 1-Imidazolyl, 1-Pyrazolyl oder 1,2,4-Triazolyl-(1) substituierten $C_1$—$C_5$ Alkylrest oder die Gruppe $—CH_2—Y—R^1$ bedeutet, wobei Y Sauerstoff oder Schwefel ist und $R^1$ einen $C_1$—$C_6$-Alkoxy-ethyl- oder einen $C_1$—$C_6$ Alkoxy-ethoxy-ethylrest bedeutet oder R einen $C_2$—$C_5$ Alkenyl-, $C_2$—$C_5$ Alkinyl-, $C_1$—$C_5$ Alkoxy-, $C_1$—$C_5$ Alkoxycarbonyl- oder einen $C_3$—$C_7$ Cycloalkylrest bedeutet.

2. Fungizid, enthaltend einen festen oder flüssigen Trägerstoff und ein N-substituiertes 2-Mehylnaphthylamid gemäß Anspruch 1.

3. Verfahren zur Herstellung eines Fungizids, dadurch gekennzeichnet, daß man einen festen oder flüssigen Trägerstoff vermischt mit einem N-substituierten 2-Methylnaphthylamid gemäß Anspruch 1.

4. Verfahren zur Bekämpfung von Pilzen, dadurch gekennzeichnet, daß man die Pilze oder die vor Pilzbefall zu schützenden Gegenstände behandelt mit einem N-substituierten 2-Methylnaphthylamid gemäß Anspruch 1.

5. Verfahren zur Herstellung eines N-substituierten 2-Methylnaphthylamids gemäß Anspruch 1, dadurch gekennzeichnet, daß man ein 2-Methylnaphthylamid der Formel II

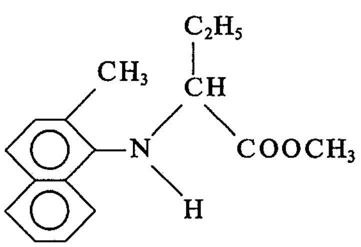

(II)

a) mit einem Säurehalogenid der Formel III

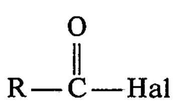

(III)

oder
b) mit einem Säureanhydrid der Formel IV

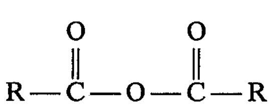

(IV)

worin
R die in Anspruch 1 angegebene Bedeutung hat und Hal Chlor oder Brom bedeutet, gegebenenfalls in Gegenwart eines Lösungsmittels, gegebenenfalls unter Zusatz einer anorganischen oder organischen Base und gegebenenfalls unter Zusatz eines Reaktionsbeschleunigers bei Temperaturen zwischen —10 und 100°C umsetzt.

6. Fungizid, enthaltend ein N-substituiertes 2-Naphthylamid definiert wie in Anspruch 1, dadurch gekennzeichnet, daß R 2-Furyl, 3- oder 5-Isothiazolyl, 2-, 4- oder 5-Thiazolyl, 3- oder 5-Isoxazolyl, 2-, 4- oder 5-Oxazolyl, 3-(1,2,4-Oxadiazolyl), 4- oder 5-(1,2,3-Thiadiazolyl), 4-(1,2,5-Thiadiazolyl), Methoxy- oder Ethoxymethyl, Methylthio- oder Ethylthiomethyl, Methoxyethyl, 1-Imidazolylmethyl,

1-Pyrazolylmethyl, 1-(1,2,4-Triazolyl)-methyl, Methyl, Ethyl, n-Propyl, Chlormethyl, Brommethyl, 1-Chlorethyl, 2-Chlorethyl, 1-Chlorpropyl, 3-Chlorpropyl, 4-Chlorbutyl, Vinyl, Propenyl, Ethinyl, Methoxy, Ethoxy, Propoxy, Methoxycarbonyl, Ethoxycarbonyl, Propoxycarbonyl, Cyclopropyl und Cyclohexyl und $R^1$-Y-Methoxyethoxy, Ethoxyethoxy, Methoxyethoxyethoxy und Butoxyethoxyethoxy bedeutet.

**Claims for the Contracting states: BE, CH, DE, FR, GB, IT, LI, NL, SE**

1. An N-substituted 2-methylnaphthylamide of the general formula I

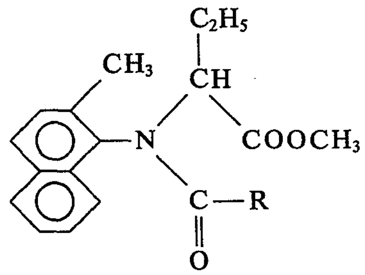

(I)

where R is an unsubstituted or methyl-substituted fur-2-yl, isoxazol-3-yl, isoxazol-4-yl, isoxazol-5-yl, oxazol-2-yl, oxazol-4-yl or oxazol-5-yl radical, or an isothiazol-3-yl, isothiazol-4-yl, isothiazol-5-yl, thiazol-2-yl, thiazol-4-yl, thiazol-5-yl, 1,2,4-oxadiazol-3-yl, 1,2,4-thiadiazol-3-yl, 1,2,4-thiadiazol-5-yl, 1,2,5-thiadiazol-3-yl or 1,3,4-thiadiazol-2-yl radical which is unsubstituted or substituted by halogen, nitro or methyl, or R is $C_1$—$C_5$-alkyl which is unsubstituted or substituted by halogen, $C_1$—$C_4$-alkoxy, $C_1$—$C_4$-alkylthio, imidazol-1-yl, pyrazol-1-yl or 1,2,4-triazol-1-yl, or R is the group —$CH_2$—Y—R, where Y is oxygen or sulfur and $R^1$ is $C_1$—$C_6$-alkoxyethyl or $C_1$—$C_6$-alkoxyethoxyethyl, or R is $C_2$—$C_5$-alkenyl, $C_2$—$C_5$-alkynyl, $C_1$—$C_5$-alkoxy, $C_1$—$C_5$-alkoxycarbonyl or $C_3$—$C_7$-cycloalkyl.

2. A fungicide containing an N-substituted 2-methylnaphthylamide as claimed in claim 1.

3. A fungicide containing an solid or liquid carrier and an N-substituted 2-methylnaphthylamide as claimed in claim 1.

4. A process for producing a fungicide, wherein a solid or liquid carrier is mixed with an N-substituted 2-methylnaphthylamide as claimed in claim 1.

5. A process for combating fungi, wherein the fungi or the objects to be protected against fungus attack are treated with an N-substituted 2-methylnaphthylamide as claimed in claim 1.

6. A process for preparing an N-substituted 2-methylnaphthylamide as claimed in claim 1, wherein a 2-methylnaphthylamine of the formula II

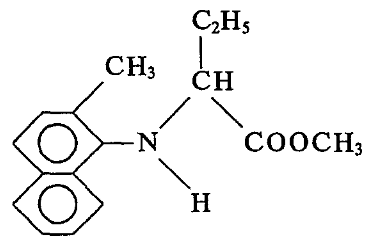

(II)

is reacted

a) with an acid halide of the formula III

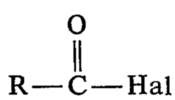

(III)

or

b) with an acid anhydride of the formula IV

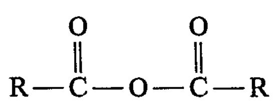

(IV)

where R has the meanings given in claim 1 and Hal is chlorine or bromine, in the presence or

absence of a solvent, with or without the addition of an inorganic or organic base, and with or without the addition of a reaction accelerator, at a temperature of from −10 to 100°C.

7. An N-substituted 2-methylnaphthylamide as claimed in claim 1, where R is fur-2-yl, isothiazol-3-yl, isothiazol-5-yl, thiazol-2-yl, thiazol-4-yl, thiazol-5-yl, isoxazol-3-yl, isoxazol-5-yl, oxazol-2-yl, oxazol-4-yl, oxazol-5-yl, 1,2,4-oxadiazol-3-yl, 1,2,3-thiadiazol-4-yl, 1,2,3-thiadiazol-5-yl, 1,2,5-thiadiazol-4-yl, methoxymethyl, ethoxymethyl, methylthiomethyl, ethylthiomethyl, methoxyethyl, imidazol-1-ylmethyl, pyrazol-1-ylmethyl, 1,2,4-triazol-1-ylmethyl, methyl, ethyl, n-propyl, chloromethyl, bromomethyl, 1-chloroethyl, 2-chloroethyl, 1-chloropropyl, 3-chloropropyl, 4-chlorobutyl, vinyl, propenyl, ethynyl, methoxy, ethoxy, propoxy, methoxycarbonyl, ethoxycarbonyl, propoxycarbonyl, cyclopropyl or cyclohexyl, and $R^1$-Y- is methoxyethoxy, ethoxyethoxy, methoxyethoxyethoxy or butoxyethoxyethoxy.

8. A fungicide containing an N-substituted 2-methylnaphthylamide as claimed in claim 1, where R is fur-2-yl, isothiazol-3-yl, isothiazol-5-yl, thiazol-2-yl, thiazol-4-yl, thiazol-5-yl, isoxazol-3-yl, isoxazol-5-yl, oxazol-2-yl, oxazol-4-yl, oxazol-5-yl, 1,2,4-oxadiazol-3-yl, 1,2,3-thiadiazol-4-yl, 1,2,3-thiadiazol-5-yl, 1,2,5-thiadiazol-4-yl, methoxymethyl, ethoxymethyl, methylthiomethyl, ethylthiomethyl, methoxyethyl, imidazol-1-yl-methyl, pyrazol-1-ylmethyl, 1,2,4-triazol-1-ylmethyl, methyl, ethyl, n-propyl, chloromethyl, bromomethyl, 1-chloroethyl, 2-chloroethyl, 1-chloropropyl, 3-chloropropyl, 4-chlorobutyl, vinyl, propenyl, ethynyl, methoxy, ethoxy, propoxy, methoxycarbonyl, ethoxycarbonyl, propoxycarbonyl, cyclopropyl or cyclohexyl, and $R^1$-Y- is methoxyethoxy, ethoxyethoxy, methoxyethoxyethoxy or butoxyethoxyethoxy.

**Claims for the Contracting state: AT**

1. A fungicide containing an N-substituted 2-methylnaphthylamide of the general formula I

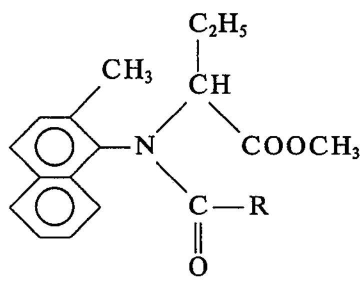

(I)

where R is an unsubstituted or methyl-substituted fur-2-yl, isoxazol-3-yl, isoxazol-4-yl, isoxazol-5-yl, oxazol-2-yl, oxazol-4-yl or oxazol-5-yl radical, or an isothiazol-3-yl, isothiazol-4-yl, isothiazol-5-yl, thiazol-2-yl, thiazol-4-yl, thiazol-5-yl, 1,2,4-oxadiazol-3-yl, 1,2,4-thiadiazol-3-yl, 1,2,4-thiadiazol-5-yl, 1,2,5-thiadiazol-3-yl or 1,3,4-thiadiazol-2-yl radical which is unsubstituted or substituted by halogen, nitro or methyl, or R is $C_1$—$C_5$-alkyl which is unsubstituted or substituted by halogen, $C_1$—$C_4$-alkoxy, $C_1$—$C_4$-alkylthio, imidazol-1-yl, pyrazol-1-yl or 1,2,4-triazol-1-yl, or R is the group $-CH_2-Y-R^1$, where Y is oxygen or sulfur and $R^1$ is $C_1$—$C_6$-alkoxyethyl or $C_1$—$C_6$-alkoxyethoxyethyl, or R is $C_2$—$C_5$-alkenyl, $C_2$—$C_5$-alkynyl, $C_1$—$C_5$-alkoxy, $C_1$—$C_5$-alkoxycarbonyl or $C_3$—$C_7$-cycloalkyl.

2. A fungicide containing a solid or liquid carrier and an N-substituted 2-methylnaphthylamide as defined in claim 1.

3. A process for producing a fungicide, wherein a solid or liquid carrier is mixed with an N-substituted 2-methylnaphthylamide as defined in claim 1.

4. A process for combating fungi, wherein the fungi or the objects to be protected against fungus attack are treated with an N-substituted 2-methylnaphthylamide as defined in claim 1.

5. A process for preparing an N-substituted 2-methylnaphthylamide as defined in claim 1, wherein a 2-methylnaphthylamine of the formula II

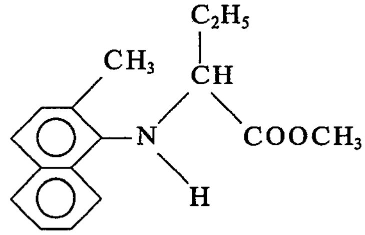

(II)

is reacted

a) with an acid halide of the formula III

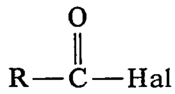

(III)

or

b) with an acid anhydride of the formula IV

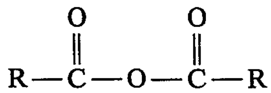

(IV)

where R has the meanings given in claim 1 and Hal is chlorine or bromine, in the presence or absence of a solvent, with or without the addition of an inorganic or organic base, and with or without the addition of a reaction accelerator, at a temperature of from −10 to 100°C.

6. A fungicide containing an N-substituted 2-methylnaphthylamide as defined in claim 1, where R is fur-2-yl, isothiazol-3-yl, isothiazol-5-yl, thiazol-2-yl, thiazol-4-yl, thiazol-5-yl, isoxazol-3-yl, isoxazol-5-yl, oxazol-2-yl, oxazol-4-yl, oxazol-5-yl, 1,2,4-oxadiazol-3-yl, 1,2,3-thiadiazol-4-yl, 1,2,3-thiadiazol-5-yl, 1,2,5-thiadiazol-4-yl, methoxymethyl, ethoxymethyl, methylthiomethyl, ethylthiomethyl, methoxyethyl, imidazol-1-ylmethyl, pyrazol-1-ylmethyl, 1,2,4-triazol-1-ylmethyl, methyl, ethyl, n-propyl, chloromethyl, bromomethyl, 1-chloroethyl, 2-chloroethyl, 1-chloropropyl, 3-chloropropyl, 4-chlorobutyl, vinyl, propenyl, ethynyl, methoxy, ethoxy, propoxy, methoxycarbonyl, ethoxycarbonyl, propoxycarbonyl, cyclopropyl or cyclohexyl, and $R^1$—Y— is methoxyethoxy, ethoxyethoxy, methoxyethoxyethoxy or butoxyethoxyethoxy.

**Revendications pour les Etats contractants: BE, CH, DE, FR, GB, IT, LI, NL, SE**

1. 2-Méthyl-naphthyl-amide N-substitué de la formule générale I

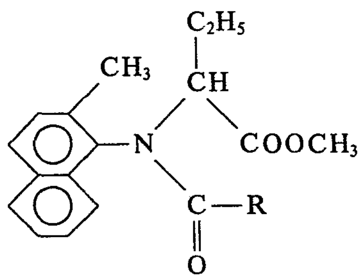

(I)

dans laquelle

R désigne un groupe 2-furyle, 3-, 4- ou 5-isoxazolyle ou 2-, 4- ou 5-oxazolyle éventuellement méthyl-substitué ou un groupe 3-, 4- ou 5-isothiazolyle, 2-, 4- ou 5-thiazolyle ou 3-(1,2,4-oxadiazolyle), 3- ou 5-(1,2,4-thiadiazolyle), 3-(1,2,5-thiadiazolyle) ou 2-(1,3,4-thiadiazolyle) éventuellement halo-, nitro- ou méthylsubstitué ou un radical alkyle en $C_1$ à $C_5$ éventuellement halo-, alcoxy (en $C_1$ à $C_4$)-, alkyl (en $C_1$ à $C_4$)-thio-, 1-imidazolyl-, 1-pyrazolyl- ou 1,2,4-triazolyl-(1)-substitué ou un groupe $-CH_2-Y-R^1$, où Y désigne un oxygène ou un soufre et $R^1$ un groupe alcoxy (en $C_1$ à $C_6$)-éthyle ou alcoxy (en $C_1$ à $C_6$)-éthoxy-éthyle,

R pouvant encore désigner un groupe alcényle en $C_2$ à $C_5$, alcynyle en $C_2$ à $C_5$, alcoxy en $C_1$ à $C_5$, alcoxy (en $C_1$ à $C_5$)-carbonyle ou cycloalkyle en $C_3$ à $C_7$.

2. Composition fongicide, contenant un 2-méthyl-naphthyl-amide N-substitué selon la revendication 1.

3. Composition fongicide, contenant un 2-méthyl-naphthyl-amide N-substitué selon la revendication 1 dans une matière support solide ou liquide.

4. Procédé de préparation d'une composition fongicide, caractérisé en ce que l'on mélange un 2-méthyl-naphthyl-amide N-substitué selon la revendication 1 avec une matière support solide ou liquide.

5. Procédé de lutte contre les champignons, caractérisé en ce que l'on traite les champignons ou les objets à protéger des champignons avec un 2-méthyl-naphthyl-amide N-substitué selon la revendication 1.

6. Procédé de préparation d'un 2-méthyl-naphthyl-amide N-substitué selon la revendication 1, caractérisé en ce que l'on fait réagir une 2-méthyl-naphthyl-amine de la formule II

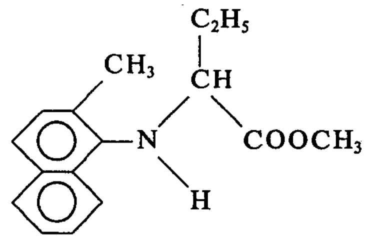

(II)

à des températures comprises entre −10 et 100°C, éventuellement dans un solvant et(ou) en présence d'une base inorganique ou organique et(ou) d'un accélérateur de la réaction, soit

a) avec un halogénure d'acide de la formule III

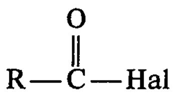

(III)

soit

b) avec un anhydride d'acide de la formule IV

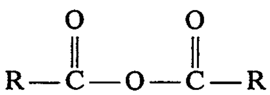

(IV)

dans lesquelles R possède la signification définie dans la revendication 1 et Hal désigne un atome de chlore ou de brome.

7. 2-Méthyl-naphthyl-amide N-substitué selon la revendication 1, caractérisé en ce que R désigne un groupe 2-furyle, 3- ou 5-isothiazolyle, 2-, 4- ou 5-thiazolyle, 3- ou 5-isoxazolyle, 2-, 4- ou 5-oxazolyle, 3-(1,2,4-oxadiazolyle), 4- ou 5-(1,2,3-thiadiazolyle), 4-(1,2,5-thiadiazolyle), méthoxy- ou éthoxy-méthyle, méthylthio- ou éthylthio-méthyle, méthoxy-éthyle, 1-imidazolyl-méthyle, 1-pyrazolyl-méthyle, 1-(1,2,4-triazolyl)-méthyle, méthyle, éthyle, n-propyle, chlorométhyle, bromo-méthyle, 1-chlor-éthyle, 2-chlor-éthyle, 1-chloro-propyle, 3-chloro-propyle, 4-chloro-butyle, vinyle, propényle, éthynyle, méthoxy, éthoxy, propoxy, méthoxy-carbonyle, éthoxy-carbonyle, propoxy-carbonyle, cyclopropyle ou cyclohexyle et $R^1$—Y— représente un groupe méthoxy-éthoxy, éthoxy-éthoxy, méthoxy-éthoxy-éthoxy ou butoxy-éthoxy-éthoxy.

8. Composition fongicide, contenant un 2-naphthylamide N-substitué tel que défini dans la revendication 1, caractérisé en ce que R désigne un groupe 2-furyle, 3- ou 5-isothiazolyle, 2-, 4- ou 5-thiazolyle, 3- ou 5-isoxazolyle, 2-, 4- ou 5-oxyzolyle, 3-(1,2,4-oxadiazolyle), 4- ou 5-(1,2,3-thiadiazolyle), 4-(1,2,5-thiadiazolyle), méthoxy- ou éthoxy-méthyle, méthylthio- ou éthylthio-méthyle, méthoxy-éthyle, 1-imidazolyl-méthyle, 1-pyrazolyl-méthyle, 1-(1,2,4-triazolyl)-méthyle, méthyle, éthyle, n-propyle, chloro-méthyle, bromo-méthyle, 1-chlor-éthyle, 2-chlor-éthyle, 1-chloro-propyle, 3-chloro-propyle, 4-chloro-butyle, vinyle, propényle, éthynyle, méthoxy, éthoxy, propoxy, méthoxy-carbonyle, éthoxy-carbonyle, propoxy-carbonyle, cyclopropyle ou cyclohexyle et $R^1$—Y— représente un groupe méthoxy-éthoxy, éthoxy-éthoxy, méthoxy-éthoxy-éthoxy ou butoxy-éthoxy-éthoxy.

**Revendications pour l'Etat contractant: AT**

1. Composition fongicide, contenant un 2-méthyl-naphthyl-amide N-substitué de la formule générale I

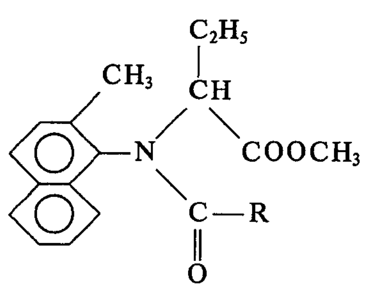

(I)

dans laquelle

R désigne un groupe 2-furyle, 3-, 4- ou 5-isoxazolyle ou 2-, 4- ou 5-oxazolyle éventuellement méthyl-substitué ou un groupe 3-, 4- ou 5-isothiazolyle, 2-, 4- ou 5-thiazolyle ou 3-(1,2,4-oxadiazolyle), 3- ou 5-(1,2,4-thiadiazolyle), 3-(1,2,5-thiadiazolyle) ou 2-(1,3,4-thiadiazolyle) éventuellement halo-, nitro- ou méthyl-substitué ou un radical alkyle en $C_1$ à $C_5$ éventuellement halo-, alcoxy (en $C_1$ à $C_4$)-, alkyl (en $C_1$ à $C_4$)-thio-, 1-imidazolyl-, 1-pyrazolyl- ou 1,2,4-triazolyl-(1)-substitué ou un groupe $-CH_2-Y-R^1$, où Y désigne un oxygène ou un soufre et $R^1$ un groupe alcoxy (en $C_1$ à $C_6$)-éthyle ou alcoxy (en $C_1$ à $C_6$)-éthoxy-éthyle,

R pouvant encore désigner un groupe alcényle en $C_2$ à $C_5$, alcynyle en $C_2$ à $C_5$, alcoxy en $C_1$ à $C_5$, alcoxy (en $C_1$ à $C_5$)-carbonyle ou cycloalkyle en $C_3$ à $C_7$.

2. Composition fongicide, contenant un 2-méthyl-naphthyl-amide N-substitué selon la revendication 1 dans une matière support solide ou liquide.

3. Procédé de préparation d'une composition fongicide, caractérisé en ce que l'on mélange un 2-méthyl-naphthyl-amide N-substitué selon la revendication 1 avec une matière support solide ou liquide.

4. Procédé de lutte contre les champignons, caractérisé en ce que l'on traite les champignons ou les objets à protéger des champignons avec un 2-méthyl-naphthyl-amide N-substitué selon la revendication 1.

5. Procédé de préparation d'un 2-méthyl-naphthyl-amide N-substitué selon la revendication 1, caractérisé en ce que l'on fait réagir une 2-méthyl-naphthyl-amine de la formule II

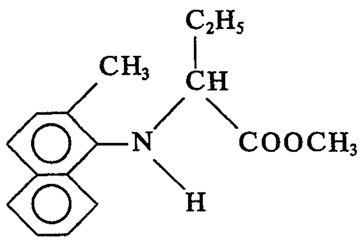

(II)

à des températures comprises entre −10 et 100°C, éventuellement dans un solvant et(ou) en présence d'une base inorganique ou organique et(ou) d'un accélérateur de la réaction, soit

a) avec un halogénure d'acide de la formule III

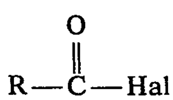

(III)

soit

b) avec un anhydride d'acide de la formule IV

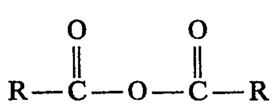

(IV)

dans lesquelles R possède la signification définie dans la revendication 1 et Hal désigne un atome de chlore ou de brome.

6. Composition fongicide, contenant un 2-naphthylamide N-substitué tel que défini dans la revendication 1, caractérisé en ce que R désigne un groupe 2-furyle, 3- ou 5-isothiazolyle, 2-, 4- ou 5-thiazolyle, 3- ou 5-isoxazolyle, 2-, 4- ou 5-oxazolyle, 3-(1,2,4-oxadiazolyle), 4- ou 5-(1,2,3-thiadiazolyle), 4-(1,2,5-thiadiazolyle), méthoxy- ou éthoxy-méthyle, méthylthio- ou éthylthio-méthyle, méthoxy-éthyle, 1-imidazolyl-méthyle, 1-pyrazolyl-méthyle, 1-(1,2,4-triazolyl)-méthyle, méthyle, éthyle, n-propyle, chloro-méthyle, bromo-méthyle, 1-chlor-éthyle, 2-chlor-éthyle, 1-chloro-propyle, 3-chloro-propyle, 4-chloro-butyle, vinyle, propényle, éthynyle, méthoxy, éthoxy, propoxy, méthoxy-carbonyle, éthoxy-carbonyle, propoxy-carbonyle, cyclopropyle ou cyclohexyle et $R^1-Y-$ représente un groupe méthoxy-éthoxy, éthoxy-éthoxy, méthoxy-éthoxy-éthoxy ou butoxy-éthoxy-éthoxy.